# EUROPEAN PATENT APPLICATION

(11) **EP 3 992 983 A1**
(43) Date of publication of application: **04.05.2022**
(21) Application number: 20204358.4
(22) Date of filing: 28.10.2020
(51) Int. Cl.: G16H 40/63, G16H 40/67, G06F 9/451, G16H 20/70, G06F 3/0481, G06F 3/0487, G09B 21/00, G06F 3/048, G09B 5/00

(54) **USER INTERFACE SYSTEM**

(71) Applicant: Koninklijke Philips N.V., 5656 AG Eindhoven (NL)
(72) Inventor: VAN GENUGTEN, Lenneke, 5656 AE Eindhoven (NL); DELLIMORE, Kiran Hamilton J., 5656 AE Eindhoven (NL); SAINI, Privender Kaur, 5656 AE Eindhoven (NL); BOS, Colin, 5656 AE Eindhoven (NL)
(74) Representative: Philips Intellectual Property & Standards

(57) **Abstract**

A user interface system and method selects a modality for transmission of data to a user, which requests actions or user input, in dependence on the user's structural preferences, contextual factors immediately preceding the intended time for transmission of data, the modality characteristics of the different possible modalities, and compliance information in respect of previous transmissions to the user.

## Description

### FIELD OF THE INVENTION

This invention relates to user interaction with a system, such as an electronic health monitoring system.

### BACKGROUND OF THE INVENTION

There are many types of electronic health monitoring systems. These may take the form of telehealth programs, coaching apps and mobile monitoring systems. In these systems, the user is often required to interact with the system. For example, a user may be required to report their symptoms, connect a device, fill out a survey, receive education, set a goal, etc.

If these interactions do not take place, the system will not have the right information or data, thereby hampering suitable actions by the system, e.g. failure to escalate when symptoms worsen. The user then will not receive the full benefit from the system, for example the user will not receive the right information or persuasive action. Therefore, it is important that users respond to system-initiated interventions.

These interventions can be implemented via several different modalities on a smartphone or tablet. These modalities relate to the carrier medium for transfer of information, such as text vs. video, interactive vs. static, silent vs with sound, etc. The assumption is that content can be provided or collected via any modality.

The best response will be achieved if the modality is most suitable to the particular user, and therefore the most efficient way to transfer content. Choosing the best modality to match user preferences will lead to more compliance, higher user satisfaction and thus higher effectiveness.

In particular, some users (e.g. patients) are not receptive to interventions in telehealth solutions, e.g. interventions for educational or information gathering (survey) purposes. For example, users may be busy or can't use their hands to navigate a screen. In order to make users more receptive, the modality of the content transfer should fit their preferences.

User preferences differ between people and between situations. For example, some patients prefer an interactive video coach because it appears to them as more personal, real, easier to understand than text only, and interactive. Others may prefer this modality only when privacy concerns, time concerns or tired eyes would not play a role.

However, other patients may prefer a text based approach because they can read it more quickly than watching a video.

Thus, people's preferences for a modality are influenced by both constant factors ('structural preferences'), such as eyesight, education level, literacy level, numeracy level, and hearing ability, as well contextual factors that fluctuate with context ('state preferences'), such as the people around, location, activity. If an intervention follows these preferences, it is more likely to be successful in transferring or gathering the information needed.

One approach is to request input from a user as to which modality they would prefer. However, this is time consuming, annoying, and indeed the wrong modality might be used to even ask for their preference.

Therefore, there is a need for a way to find the most appropriate user-fitting modality, which leads to the best response, and therefore to the most efficient content transfer while accounting for both structural and state user preferences.

### SUMMARY OF THE INVENTION

The invention is defined by the claims.

According to examples in accordance with an aspect of the invention, there is provided a user interface system, comprising:
an output for providing content to a user and requesting user input and/or actions;
an input for receiving input from a user;
a database structure storing:
   first information about a users' structural preferences resulting from their aptitudes or preference for different communications modalities;
   second information about the contextual factors that will temporarily influence or override the user's preferences;
   different modality characteristics; and
   compliance information in respect of previously requested user input and/or actions; and
a modality selection unit, which is adapted to select a modality for transmission of data to the user in dependence on the user's structural preferences, the contextual factors immediately preceding the intended time for transmission of data, the compliance information and the modality characteristics.

This system provides content to a user as well as requiring compliance with certain actions and requiring input from the user at various times. This compliance with actions is reported back to the system via the input and/or optionally via sensors. The mere existence of an input may itself indicate compliance with a request for user input. Compliance with actions may instead require the user to complete the action and separately communicate that the action has been completed. The aim of the system is to improve compliance with the requested actions and compliance with the request for input, and thereby improve a service (e.g. telehealth, coaching, education, information collection, etc.) provided to the user.

The modality selection unit produces an output, based on processing of the various data sources in the database structure, using a suitable modality. The selected modality best matches the current situation of the user for the next planned output to the user i.e. user intervention, but also taking into account the historical compliance.

The invention in particular addresses a problem of low effectiveness of interventions due to poor user compliance, and low user satisfaction due to poor content transfer between a system and the user. In particular, the system chooses a modality which is most appropriate for a user based on the user's fixed structural and contextual state preferences.

The system may further facilitate or optimize the user environment to make the user more likely to be compliant with the selected modality. This may involve selecting a suitable volume, or screen brightness, or pausing other devices which are providing content to the user, etc.

The first information may comprise information about one or more of:
the eyesight of the user;
the numeracy level of the user;
the education level of the user;
the cognitive learning style of the user;
the reading literacy level of the user;
the digital literacy level of the user;
the hearing ability of the user;
a preferred modality type of the user; and
a preferred device type of the user.

This information enables a best modality to be selected to match a user's aptitudes or preferences (e.g. phone vs tablet vs PC).

The second information may comprise information about one or more of:
the proximity of the user to other people;
the user location; and
an activity being performed by the user.

These enable different situations to be identified which the user may be experiencing.

The user interface system may comprise an input for receiving location information from a portable device of the user.

Information may for example be gathered via a user's portable or wearable device (e.g. a smart phone, tablet or smart watch).

The modality characteristics may distinguish between:
sound emitting and silent; or
text based and graphics based; or
interactive and one-way; or
compact and detailed; or
time sensitive and time insensitive;
privacy sensitive and privacy non-sensitive.

Thus, different modalities have different characteristics which will be suitable in different situations.

The compliance information may express a relationship between (i) the modality type and associated contextual factors for historical requested user input and/or actions and (ii) the associated compliance rate.

Thus, the historical information takes account of the type of modality used in different contexts and the corresponding compliance rate. Thus, the system may adapt and evolve over time to increase the compliance rate.

The compliance information may comprise a response time to a request for user action or user input.

For example, for a user to be compliant to a requested action or input, they should in general take action within a limited time frame (after the intervention is sent) and the user context should not change, otherwise the selected modality is no longer appropriate.

The compliance may be determined in an automatic objective manner via sensors and/or via manual user input. In some cases, the manual input is itself the compliance.

The database structure may further comprise third information relating to the the user's personal activity agenda. This provides another way to know the activity of the user, in addition to sensing in real time.

The database structure may further comprise information concerning the user's preferred modality for different activities of the personal activity agenda.

The user may have certain preferences for certain activities, such as not being disturbed at all e.g. during meditation.

The user interface system may further comprise a physiological sensor arrangement for providing at least part of the second information.

Vital signs measurements may be used to help guide the selection of the most suitable modality. Examples of sensed information include physical activity, heart rate, skin conductance and blood pressure. This type of information is often collected with a smart watch, but can also be obtained from others devices, e.g. a HR monitor.

For example, someone walking or cycling could listen to a podcast or short message, but not watch a video or fill out a survey. Similarly, if someone feels agitated, it might not be the right moment to share a long message with them. Instead, it should be as short and to the point as possible.

The user interface system for example comprises a health monitoring system, wherein the output is for providing health advisory content to a user and requesting user input and/or actions.

The invention also provides a user interface method, comprising:
selecting a modality for transmission of data to a user in dependence on:
   users' structural preferences resulting from their aptitudes or preferences for different communications modalities;
   contextual factors that will temporarily influence or override the user's preferences, immediately preceding the intended time for transmission of data;
   different modality characteristics; and
   compliance information in respect of previously requested user input and/or actions;
providing content to a user using the selected modality, the content requesting user input and/or actions; and
receiving input from a user relating to compliance with the requested user input and/or actions.

The method may comprise:
deriving the user structural preferences from one or more of:
   the eyesight of the user;
   the numeracy level of the user;
   the education level of the user;
   the cognitive learning style of the user;
   the reading literacy level of the user;
   a digital literacy level of the user;
   the hearing ability of the user;
   a preferred modality type of the user; and
   a preferred device type of the user; and
the contextual factors comprise one or more of:
   the proximity of the user to other people;
   the user location; and
   an activity being performed by the user.

The compliance information may express a relationship between (i) the modality type and associated contextual factors for historical requested user input and/or actions and (ii) the associated compliance rate.

The method is preferably used for providing health advisory content to a user and requesting user input and/or actions.

The method of the invention may be implemented in software.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a user interface system; and
Figure 2 shows a user interface method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a user interface system and method which selects a modality for transmission of data to a user, which requests actions or user input, in dependence on the user's structural preferences, contextual factors immediately preceding the intended time for transmission of data, the modality characteristics of the different possible modalities, and compliance information in respect of previous transmissions to the user.

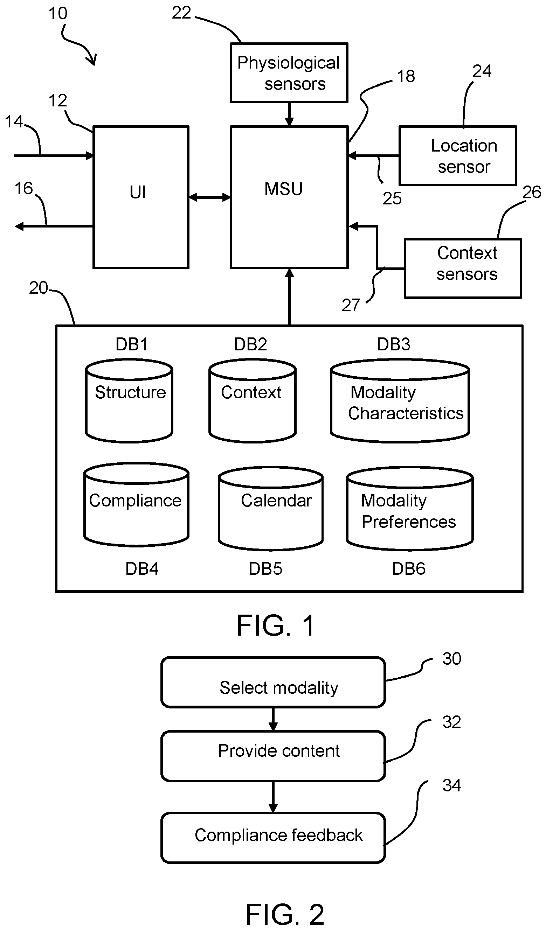

Figure 1 shows a user interface system 10.

The system comprises a user interface 12 and a processor 18 which functions as a modality selection unit (MSU).

The user interface is a device which enables user input (to input 14) and provides user output (at output 16). The user interface for example comprises a device with a display and speakers for output, and a microphone and touch screen for input. Of course, other input and output modalities may be used, such as a vibration output and an input based on a position, orientation, gesture or a movement pattern applied to the user interface.

The user interface may be implemented by a mobile phone of the user.

The processor may be the processor of such a mobile phone, or it may be a remote processor with which the mobile phone (or other portable device held by the user) communicates.

The output 16 provides content to a user and also requests user input and/or actions. This input may a response to questions (i.e. a questionnaire) or confirmation that requested actions have been taken (e.g. taking medication or performing exercise).

The system has a database structure 20 which stores various types of information. The various data types are described below as being in different databases, but this is for convenience only. There may be only a single data structure.

A first database DB 1 stores first information about a users' structural preferences relevant to communications to be sent to the user. These structural preferences result from their aptitudes or preference for different communications modalities.

The first information may comprise information about one or more of:
the eyesight of the user;
the numeracy level of the user;
the education level of the user;
the cognitive learning style of the user;
the reading literacy level of the user;
the digital literacy level of the user;
the hearing ability of the user;
a preferred modality type of the user; and
a preferred device type of the user.

This information enables a best modality to be selected to match a user's aptitudes or preferences (e.g. phone vs tablet vs PC). Other examples are preferred social media, preferred method of keeping notes and reminders (e.g. text, calendar, voice, particular software).

The first information in the first database DB1 is for example obtained from one-time surveys or questionnaires filled out by the user, or from historical user information in an electronic record (e.g., an electronic medical record or a user device profile which may contain user device settings including font size and sound volume which can be used to infer structural preferences).

A second database DB2 stores second information about the contextual factors that will temporarily influence or override the user's preferences. These contextual factors are for example determined by a location sensor 24 (which provides an input 25 to the processor 18) and optionally additional context sensors 26 (which provide an input 27 to the processor 18) and they relate to real time conditions that influencing the user's ability to receive communications with different modalities.

The location sensor is for example the GPS sensor of the mobile phone. The other context sensors for example may use a microphone (of the mobile phone) to detect noise levels and proximity to other people, and acceleration and/or gyroscope sensors (of the mobile phone) to detect movements and orientation. The camera of a mobile phone can also provide other information relevant to context.

The second information for example comprises information about one or more of:
the proximity of the user to other people;
the user location; and
an activity being performed by the user.

The second information thereby enables different user situations to be identified. Other examples are environmental factors (e.g., background noise and light level). The activity of the user may for example be walking, cycling, driving etc.

The second information in the second database DB2 is for example acquired from sensors in wearable and portable devices in the vicinity of the user such as a smartphone, smart watch, tablet, etc. as mentioned above.

Proximity to other people may for example be determined by Bluetooth and Wi-Fi signatures. Environmental factors (e.g., background noise level and light level) may be determined from the microphone and light sensors. This may also include information from phone or device usage. For example, it may be determined if the user is currently using social media (and which medium), watching videos on YouTube^{™}, using a car Bluetooth^{™} kit to have conversations, or using WhatsApp^{™} for chatting. This information indicates which modality is already in use and therefore might be preferred at this moment.

A third database DB3 stores information which classifies the different modalities for communication according to the different modality characteristics.

The modality characteristics may distinguish between:
sound emitting and silent; or
text based and graphics based; or
interactive and one-way; or
compact and detailed; or
time sensitive and time insensitive;
privacy sensitive and privacy non-sensitive.

Thus, different modalities have different characteristics which will be suitable in different situations or equally importantly will not be suitable in some situations.

A fourth database DB4 stores historical compliance information in respect of previously requested user input and/or actions. This is used to judge the previous success of different communication modalities in different contextual situations. Thus, for a given historical combination of modality type and contextual factors, an associated compliance rate can be determined. The system may thus adapt and evolve over time to increase the compliance rate.

The compliance information in the fourth database DB4 may comprise a response time to a request for user action or user input. For example, for a user to be compliant to a requested action or input, they should in general take action within a limited time frame (after the intervention is sent) and the user context should not change, otherwise the selected modality is no longer appropriate.

The compliance may be determined in an automatic objective manner via sensors and/or via manual user input.

The use of the compliance information means the system has learned from the users' response to certain modalities in certain contexts in the past. The historical compliance and effectiveness of modalities is thus also used to help guide the selection of the most suitable modality while accounting for state and structural preferences, given a certain context.

The compliance information for example includes information about the response time to open an intervention prompt (e.g. a push message), the time to take the next step (e.g. to open a video, to fill out a survey), the time to complete the survey, etc.

Storing this compliance data allows analysis of not only if a modality was successful, but also in which context it was successful. The compliance information thus enables the success of a modality to be judged taking into account the context in which the intervention was sent with that modality. For a user to be compliant, they should in general take action within a limited time frame (after the intervention is sent) and the user context should not change, otherwise the selected modality is no longer appropriate.

Thus, it is also possible to resend an intervention with a new modality if the user context has changed (as can be detected using the context sensors) before there has been compliance.

A fifth (optional) database DB5 stores information relating to the the user's personal activity agenda, i.e. their personal calendar. The expected user activities and hence contexts can thus be known in advance. Information may be stored in a sixth (optional) database DB6 concerning the user's preferred modality for the different activities they undertake.

The use of the calendar information additionally allows intelligent scheduling and postponing of interventions, around the daily planned work and social activities. The calendar items may include information about location and other participants, e.g. sports, travelling and social activities. This will allow planning of modalities even better (e.g. 'saving' a podcast until user is driving to another location or going out for a run).

The user interface is further shown having physiological sensors 22. These for example provide further contextual information based on the physiological state of the user. Vital signs measurements made by the physiological sensors may be used to help guide the selection of the most suitable modality. Examples of sensed information include physical activity, heart rate, skin conductance and blood pressure. This type of information is often collected with a smart watch, for example with a PPG sensor, but can also be obtained from others devices, e.g. a HR monitor.

From this vital signs data, it is possible to learn whether people are at rest or currently being active, aroused/ agitated or feeling relaxed, etc. For example, someone walking or cycling could listen to a podcast or short message, but not watch a video or fill out a survey. Similarly, if someone feels agitated, it might not be the right moment to share a long message with them. Instead, it should be as short and to the point as possible.

The processor 20 selects a modality for transmission of data to the user in dependence (at least) on the data in the database structure, in particular the user's structural preferences, the contextual factors immediately preceding the intended time for transmission of data, the compliance information and the modality characteristics.

This system provides content to a user as well as requiring compliance with certain actions and requiring input from the user at various times. This compliance with actions is reported back to the system via the input. The aim of the system is to improve compliance with the requested actions and compliance with the request for input, and thereby improve a service (e.g. telehealth, coaching, education, information collection, etc.) provided to the user.

The modality selected by the system best matches the current situation of the user for the next planned output to the user i.e. user intervention, but also taking into account the historical compliance. The invention in particular addresses a problem of low effectiveness of interventions due to poor user compliance, and low user satisfaction due to poor content transfer between a system and the user. In particular, the system chooses a modality which is most appropriate for a user based on the user's fixed structural and contextual state preferences.

Examples of different modality are:
written text to be read on a display;
a graphical output on a display;
a sound to be played back.

These may be provided as an email, a text, a communication over social media, a webpage, a reminder in a calendar etc. The information may be provided directly as a file or as a link to a source of information to accessed.

The user interface system is of particular interest for a health monitoring system, wherein the output is for providing health advisory content to a user and requesting user input and/or actions. Some examples of possible uses of the system will now be presented.

A first scenario may be considered in which a non-time-sensitive educational intervention is planned for a user, with a preference for a smartphone device and with poor hearing (these are the structural preferences) while the user is at a cafe (as determined by GPS), with loud background music and good lighting (as determined by the microphone and light sensors), with several other people in close proximity (as measured by Bluetooth and Wi-Fi signatures).

In this case the processor, based on the structural and context preferences, will determine the current user preferences, which will then be compared with the modality characteristics stored in the third database DB3 to select best intervention modalities for the user at that moment.

A set of possible modalities may be selected, such as the three most suitable intervention modalities. In this situation, they may be:
i) send a short, non-interactive text message to the user on their smartphone device;
ii) postpone the intervention to a later moment when the user is alone; and
iii) reschedule the intervention to the following day.

In another example, with an at-home user who has different structural preferences (e.g., poor eyesight and prefers a tablet), the processor may select a different set for the top three most suitable intervention modalities:
i) share a video with the education;
ii) share a podcast with the information; and
iii) share a very short text version, with optional audio included.

In another example, a scenario may be considered in which a time-sensitive educational intervention is to be delivered to a user, who has a preference for a tablet device, is driving alone in a car with their smartphone connected to the Bluetooth speaker while listening to music.

The top three most suitable modalities selected by the processor may be:
i) audio delivery of the intervention to the user, but not including video or visuals;
ii) ask the user with one question when would be a good time to snooze the intervention;
iii) postpone the intervention to a later moment when the user is not driving and has access to a tablet.

As explained above, historical compliance data is used. Some ways of collecting compliance data are discussed below.

A first example is a request to complete a surveys or quiz. The user submits answers to a health status survey or educational quiz to check if learning content has been understood by the patient. The user input itself confirms compliance and the time delay can be recorded as a level of compliance.

A second example is playing of an educational audio, video or podcast till the end. The complete playing itself confirms compliance.

A third example is providing an educational text message. The compliance can be determined by monitoring whether the user opened the message and scrolled to the bottom of message. Thus, there can be automatic monitoring from the device.

A fourth example is a providing a reminder to refill/collect medication. It can be monitored if the user collects the medication on time as compliance based on an automatic input from the user's medical record.

A fifth example is instructing a user to take medication using a smart nebulizer. The nebulizer can automatically confirm that the device has been used correctly and when (e.g., duration of inhalation, sound).

In the examples above, the system reacts to the current context of the user, and selects a modality accordingly, taking into account previous compliance. In a modification, the system may further facilitate or optimize the user environment to make the user more likely to be compliant with the selected modality.

A first example is that the volume of background noise (e.g. from a TV or radio etc.) may be automatically lowered or turned off when the intervention is sent to the user, in order to allow the user to focus on the intervention, e.g., an educational podcast or video.

A second example is that if the patient is driving in a car and playing music, the music may be paused in order for an audio message to be communicated to the user.

These examples thus involve adapting other devices which are providing content to the user.

A third example is that the device screen illumination may be adjusted (e.g., brightened) in order to allow the patient to more easily read an intervention sent by text message. A fourth example is that the volume of a video intervention may automatically be increased slightly to allow the patient to hear better in a setting in which the background noise is loud and not controllable, such as a cafe. A fifth example is that the text may be adjusted (e.g., the font size is increased) to make an educational text message easier to read.

These examples thus involve adapting specifics of the selected modality in order to best match the context.

Figure 2 shows a user interface method.

In step 30, a modality for transmission of data to a user is selected. This is in dependence on the users' structural preferences, the contextual factors, the different modality characteristics and the compliance information.

In step 32, content is provided to the user using the selected modality, the content requesting user input and/or actions.

In step 34 input is received from a user relating to compliance with the requested user input and/or actions.

The invention may be applied in any user facing application, either in the home or at work or in a hospital. Examples include self-management applications for COPD patients, when they collect a daily symptom survey or provide information, a pregnancy monitoring app for pregnant women, when they give information about pregnancy. The invention may be applied to non-medical fields such as for user interfaces to domestic appliances (using voice or video messages, so people don't have to use their hands while cooking)

As discussed above, the system makes use of a processor to perform the data processing. The skilled person would be readily capable of developing a processor for carrying out any herein described method. Each step of a flow chart may represent a different action performed by a processor, and may be performed by a respective module of the processor.

The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

**1.** A user interface system (10), comprising:
an output (16) for providing content to a user and requesting user input and/or actions;
an input (14) for receiving input from a user;
a database structure (20) storing:
first information (DB1) about a users' structural preferences resulting from their aptitudes or preference for different communications modalities;
second information (DB2) about the contextual factors that will temporarily influence or override the user's preferences;
different modality characteristics (DB3); and
compliance information (DB4) in respect of previously requested user input and/or actions; and
a modality selection unit (18), which is adapted to select a modality for transmission of data to the user in dependence on the user's structural preferences, the contextual factors immediately preceding the intended time for transmission of data, the compliance information and the modality characteristics.

**2.** The user interface system of claim 1, wherein the first information (DB1) comprises information about one or more of:
the eyesight of the user;
the numeracy level of the user;
the education level of the user;
the cognitive learning style of the user;
the reading literacy level of the user;
the digital literacy level of the user;
the hearing ability of the user;
a preferred modality type of the user; and
a preferred device type of the user.

**3.** The user interface system of claim 1 or 2, wherein the second information (DB2) comprises information about one or more of:
the proximity of the user to other people;
the user location; and
an activity being performed by the user.

**4.** The user interface system of claim 3, comprising an input (25) for receiving location information from a portable device of the user.

**5.** The user interface system of any one of claims 1 to 4, wherein the modality characteristics (DB3) distinguish between:
sound emitting and silent; or
text based and graphics based; or
interactive and one-way; or
compact and detailed; or
time sensitive and time insensitive;
privacy sensitive and privacy non-sensitive.

**5.** The user interface system of any one of claims 1 to 4, wherein the compliance information (DB4) expresses a relationship between (i) the modality type and associated contextual factors for historical requested user input and/or actions and (ii) the associated compliance rate.

**6.** The user interface system of any claim 5, wherein the compliance information (DB4) comprises a response time to a request for user action or user input.

**7.** The user interface system of any one of claims 1 to 6, wherein the database structure (20) further comprises third information (DB5) relating to the user's personal activity agenda.

**8.** The user interface system of any one of claims 1 to 7, wherein the database structure further comprises information (DB6) concerning the user's preferred modality for different activities of the personal activity agenda.

**9.** The user interface system of any one of claims 1 to 8, further comprising a physiological sensor arrangement (22) for providing at least part of the second information.

**10.** The user interface system of any one of claims 1 to 9 comprising a health monitoring system, wherein the output is for providing health advisory content to a user and requesting user input and/or actions.

**11.** A user interface method, comprising:
selecting a modality for transmission of data to a user in dependence on:
users' structural preferences resulting from their aptitudes or preference for different communications modalities;
contextual factors that will temporarily influence or override the user's preferences, immediately preceding the intended time for transmission of data;
different modality characteristics; and
compliance information in respect of previously requested user input and/or actions;
providing content to a user using the selected modality, the content requesting user input and/or actions; and
receiving input from a user relating to compliance with the requested user input and/or actions.

**12.** The method of claim 11, comprising:
deriving the user structural preferences from one or more of:
the eyesight of the user;
the numeracy level of the user;
the education level of the user;
the cognitive learning style of the user;
the reading literacy level of the user;
the digital literacy level of the user;
the hearing ability of the user;
a preferred modality type of the user; and
a preferred device type of the user; and
the contextual factors comprise one ore more of:
the proximity of the user to other people;
the user location; and
an activity being performed by the user.

**13.** The method of any one of claims 11 to 12, wherein the compliance information expresses a relationship between (i) the modality type and associated contextual factors for historical requested user input and/or actions and (ii) the associated compliance rate.

**14.** The method of any one of claims 11 to 13 for providing health advisory content to a user and requesting user input and/or actions.

**15.** A computer program comprising computer program code means which is adapted, when said program is run on a computer, to implement the method of any one of claims 11 to 14.
